# EUROPEAN PATENT APPLICATION

(11) **EP 2 154 146 A1**
(43) Date of publication of application: **17.02.2010**
(21) Application number: 08761510.0
(22) Date of filing: 17.04.2008
(51) Int. Cl.: C07K 14/01, A61K 39/39, A61P 37/04

(54) **USE OF AFRICAN SWINE PEST HAEMOGLUTININ AS AN ADJUVANT**

(30) Priority: 17.04.2007 ES 200701023
(71) Applicant: Centre De Recerca En Sanitat Animal (CRESA), 08193 Bellaterra - Barcelona (ES)
(72) Inventor: MARQUES ARGUILAGET, Jordi, E-08193 Bellaterra - Barcelona (ES); PEREZ MARTIN, Eva, E-08193 Bellaterra - Barcelona (ES); FERNANDEZ-BORGES, Natalia, E-08193 Bellaterra - Barcelona (ES); RODRIGUEZ GONZALEZ, Fernando, E-08193 Bellaterra - Barcelona (ES)
(74) Representative: ABG Patentes, S.L.
(86) International application number: PCT/ES2008/000264
(87) International publication number: WO 2008/129103

(57) **Abstract**

The invention generally relates to the use of the hemagglutinin (HA) of African swine fever virus (ASFV) as an adjuvant to enhance the immune response against an antigen in a subject. The invention provides a gene construct comprising all or part of the encoding sequence of said HA fused to the encoding sequence of an antigen. The invention is applicable in human and animal health.

## Description

### Field of the Invention

The invention generally relates to the use of the hemagglutinin (HA) of African swine fever virus (ASFV) as an adjuvant to enhance the immune response against an antigen in a subject. The invention also relates to gene constructs completely or partially comprising the encoding sequence of said viral hemagglutinin fused to the encoding sequence of an antigen, and to the applications thereof.

### Background of the Invention

Vaccines form a successful and widely accepted method in the prevention and treatment of infectious diseases. They are effective and do not induce resistance against antibiotics of the target pathogen or affect the normal flora of the host. In many cases, such as when an antiviral immunity is induced, vaccines can prevent a disease for which there is no viable palliative curative treatment.

Vaccines work such that they make the immune system trigger a response against an agent, or antigen, typically an infectious organism or a portion thereof which is introduced in the body in a non-infectious or -pathogenic form. Once the immune system has been sensitized with said agent, a subsequent exposure over time to said organism results in a rapid and robust immune response capable to destroy the pathogen before it can multiply and infect enough cells in the host organism so as to cause symptoms of the disease.

The agent or antigen used to develop the immune response can be the microorganism causing the disease in its complete form in a less infectious state, known as attenuated microorganism, or, in some cases, components of the organism (in the form of subunits), such as carbohydrates, proteins or peptides which represent several structural components of the organism. Likewise, among new technologies, it is worth emphasizing immunization with recombinant plasmids of transitory expression or DNA vaccines.

DNA vaccines are based on immunization with a plasmid containing the genetic information of one or several genes encoding immunogenic proteins of one or more determined pathogens, against which pathogen or pathogens a protective immune response in the vaccinated subject is to be induced in the vaccinated subject. Said plasmid acts like a vector carrying genetic information of interest into the cells of the host for the expression, processing and presentation thereof to the immune system, in what would be an imitation of what occurs during infection with the complete pathogen. The generated humoral or cellular immune response prepares the vaccinated subject to counteract an infection with the pathogen, such that its prophylactic use constitutes a potential tool to be applied, especially in those diseases requiring both branches of the immune response, such as those caused by viruses.

The use of monophosphoryl lipid A or QS-21 saponin as chemical adjuvants in DNA vaccination is known. The use of a cationic liposome formulation (DOTAP) in DNA vaccination has also been described (Etchart et al. J. Gen. Virol. 1997, 78:1577-1580), an adjuvant effect being observed when it was administered orally, but not when it was administered intranasally. DOTAP has also been used in DNA vaccines encoding the hemagglutinin of the influenza virus; however, its administration inhibits the immune response.

The use of carriers capable of specifically directing the vaccine antigen to the antigen-presenting cells (APCs), i.e., to the "smite" of induction of an immune response in the organism, has several advantages with respect to the classic adjuvants used up to now. On one hand, they reduce the toxicity by activating in a very specific, local and controlled manner only the cells expressing the specific receptor recognized by the carrier, those APCs on the other hand being responsible for presenting the vaccine antigen to the immune system (Lew AM, Brady BJ, Boyle BJ. Site-directed immune responses in DNA vaccines encoding ligand-antigen fusions. Vaccine. 2000 Feb 25;18(16):1681-5). A good example of the success of this type of strategy comes from the use of the molecule CTLA4 as an adjuvant in DNA vaccination protocols, initially described at the end of the 1990s (Boyle JS, Brady JL, Lew AM. Enhanced responses to a DNA vaccine encoding a fusion antigen that is directed to sites of immune induction. Nature. 1998 Mar 26; 392(6674):408-11). Despite the fact that the effectiveness of the CTLA4 molecule for directing the vaccine antigens to the APCs expressing the receptors thereof (B7.1 and B7.2) has been demonstrated, the use of this T-lymphocyte surface antigen as a carrier could have undesired side effects, such as the non-specific blocking of the T response. In fact, the same molecule which is used in DNA vaccination protocols as an adjuvant and antigen carrier has recently been proposed as an immunosuppressant in transplant protocols (Li S, Salgar SK, Thanikachalam M, Murdock AD, Gammie JS, Demetris AJ, Zeevi A, Pham SM. CTLA4-Ig-based conditioning regimen to induce tolerance to cardiac allografts. J Surg Res. 2006 Dec; 136(2):238-46. Epub 2006 Oct 13) or to treat autoimmune processes (Pollard LC. Inhibiting costimulatory activation of T cells: a viable treatment option for rheumatoid arthritis? Drugs. 2007; 67(1):1-9).

Therefore there is still a need to identify new carriers capable of specifically driving the vaccine antigen to the APCs for the purpose of enhancing the immune response against an antigen in a subject.

### Summary of the Invention

The invention relates to a new vaccine strategy which allows enhancing both the humoral response (antibodies) and the induced cellular response after vaccination.

The inventors have surprisingly found that the hemagglutinin (HA) of African swine fever virus (ASFV) enhances the immune response induced against an antigen in a subject, both the humoral response and the induced cellular response after vaccination with an antigen, therefore having an adjuvant effect when it is used in DNA vaccination. Said HA of ASFV seems to have the capacity to specifically drive an antigen to professional APCs (mainly macrophages and dendritic cells), thus enhancing the immune response induced against an antigen in a subject. Although the inventors do not wish to be bound by any theory, it is thought that the capacity of said HA of ASFV to direct antigens specifically to professional APCs is due to the fact that said protein has a high homology to the leukocyte surface antigen CD2 which, as is known, has receptors in APCs.

In the assays conducted by the inventors, DNA vaccines have been developed by fusing a polynucleotide encoding a determined antigen and a polynucleotide encoding the soluble fraction of the HA (sHA) of ASFV, and the immune response in pigs immunized with such DNA vaccines has been evaluated (Example 1), observing that after the immunization of pigs with said DNA vaccines, a high immune response against said antigen occurs, being it much higher than that observed in control animals, which clearly shows that the antibody response and the cellular response specifically directed against an antigen after the DNA vaccination is enhanced if said antigen is fused to said HA of ASFV or to a functionally equivalent variant thereof.

Therefore, in one aspect, the invention relates to a gene construct comprising (a) a polynucleotide (A) selected from (i) a polynucleotide comprising the nucleotide sequence encoding the hemagglutinin (HA) of African swine fever virus (ASFV); (ii) a functionally equivalent fragment of said polynucleotide defined in (i); and (iii) a functionally equivalent variant of said polynucleotide defined in (i) showing a minimum identity of 80% with respect to the encoding sequence of the HA of ASFV; and (b) a polynucleotide (B) comprising the nucleotide sequence encoding an antigen.

In another aspect, the invention relates to a vector comprising said gene construct.

In another aspect, the invention relates to a fusion protein obtainable by the expression of said gene construct or of the encoding sequence contained in said vector. The process for producing the fusion protein constitutes an additional aspect of this invention.

In another aspect, the invention relates to a host cell containing said gene construct, said vector or said fusion protein.

In another aspect, the invention relates to a pharmaceutical composition comprising a therapeutically effective amount of said gene construct, or of said vector, or of said fusion protein, or of said host cell together with a pharmaceutically acceptable carrier.

In another aspect, the invention relates to the use of said gene construct, or of said vector, or of said fusion protein, or of said host cell in the preparation of a pharmaceutical composition for the prevention and/or the treatment of a disease caused by an organism expressing or containing said antigen.

In another aspect, the invention relates to a method for enhancing the immune response against an antigen comprising administering to a subject said gene construct in which said polynucleotide (B) comprises the nucleotide sequence encoding said antigen, or a vector comprising said gene construct, or a fusion protein such as that previously mentioned comprising said antigen.

In another aspect, the invention relates to the use of the HA of ASFV, or of a functionally equivalent fragment thereof, or of a functionally equivalent variant thereof showing a minimum identity of 80% with respect to the amino acid sequence of the HA of ASFV, as an adjuvant in the preparation of a pharmaceutical composition, such as a vaccine or immunotherapeutic composition comprising an antigen.

In another aspect, the invention relates to the use of the HA of ASFV, or of a functionally equivalent fragment thereof, or of a functionally equivalent variant thereof showing a minimum identity of 80% with respect to the amino acid sequence of the HA of ASFV, as an adjuvant in the preparation of a composition for enhancing the immune response against an antigen jointly administered with said immune response-enhancing composition.

In another aspect, the invention relates to an antibody or fragment thereof recognizing said fusion protein, to pharmaceutical compositions comprising said antibody or fragment thereof recognizing said fusion protein, and to the use of said antibody or fragment thereof recognizing said fusion protein in the preparation of a pharmaceutical composition for the prevention and/or treatment of a disease caused by an organism containing the antigen present in said fusion protein.

In another aspect, the invention relates to an isolated cell of the immune system with the capacity to recognize said fusion protein, to pharmaceutical compositions comprising said cell of the immune system, and to the use of said cell of the immune system in the preparation of a pharmaceutical composition for the prevention and/or treatment of a disease caused by an organism containing the antigen present in said fusion protein.

### Brief Description of the Drawings

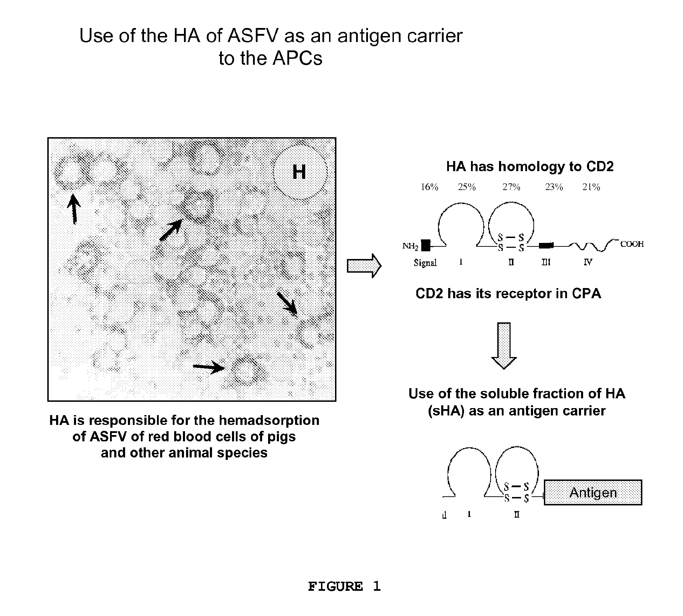

Figure 1 shows how the erythrocytes are able to bind to the cells expressing the HA of ASFV, forming a typical rosette image; it furthermore schematically shows the strategy for the fusion of the soluble fraction of the HA (sHA) of ASFV to an antigen, paying particular attention to the domains with homology to the leukocyte surface antigen CD2.

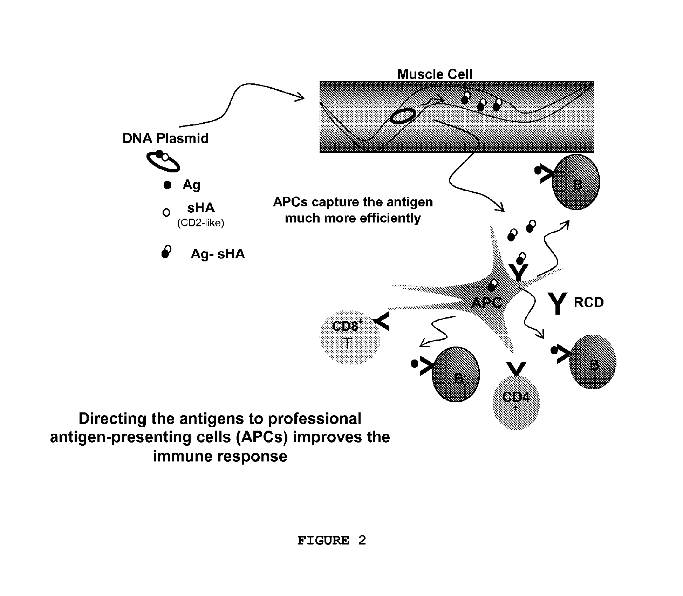

Figure 2 schematically shows the foundation of this invention, based on enhancing the induced immune response against an antigen by means of its fusion to the sHA fraction of ASFV, making use of its possible capacity of "driving" antigens towards the APCs.

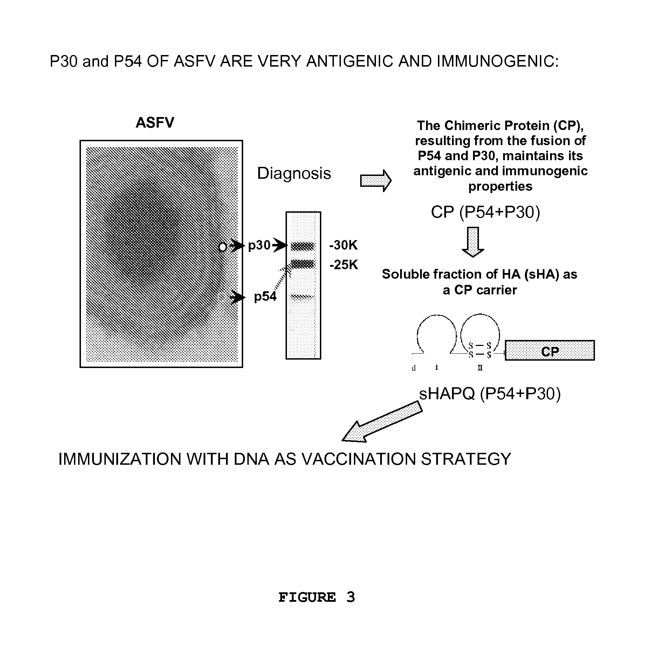

Figure 3 schematically shows the fusion protein resulting from the fusion of (i) p54 and p30 proteins of ASFV forming the fusion protein identified in this description as chimeric protein or CP, and (ii) the sHA fraction of ASFV; said p54 and p30 proteins are two immunorelevant antigens of ASFV, which justifies their selection as a vaccine antigens.

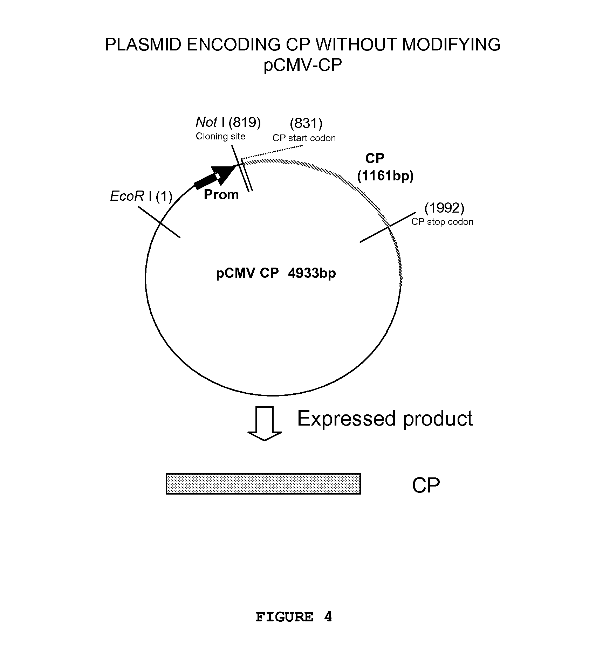

Figure 4 is a schematic depiction of plasmid pCMV-CP and the expressed product (CP), i.e., the fusion protein formed by the p54 and p30 proteins of ASFV.

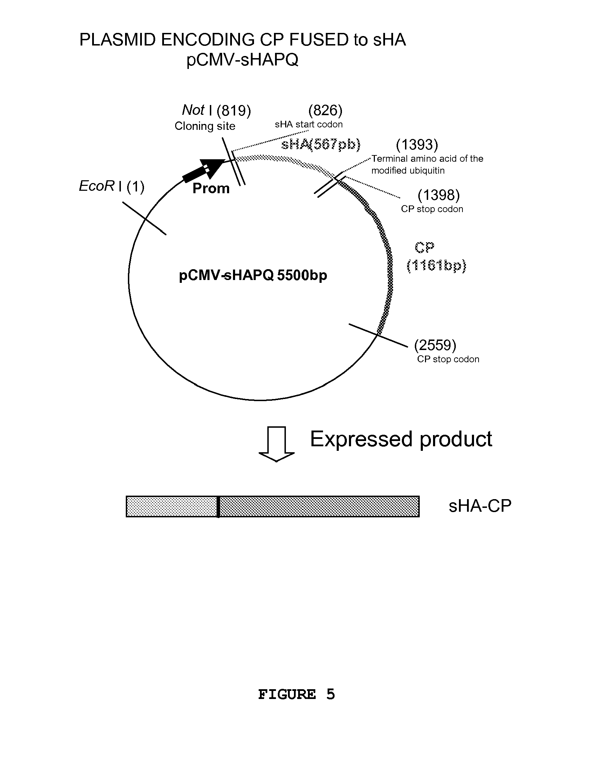

Figure 5 is a schematic depiction of plasmid pCMV-sHAPQ and the expressed product (sHAPQ), i.e., the fusion protein resulting from the fusion of (i) the p54 and p30 proteins of ASFV (CP) and (ii) the sHA fraction of ASFV.

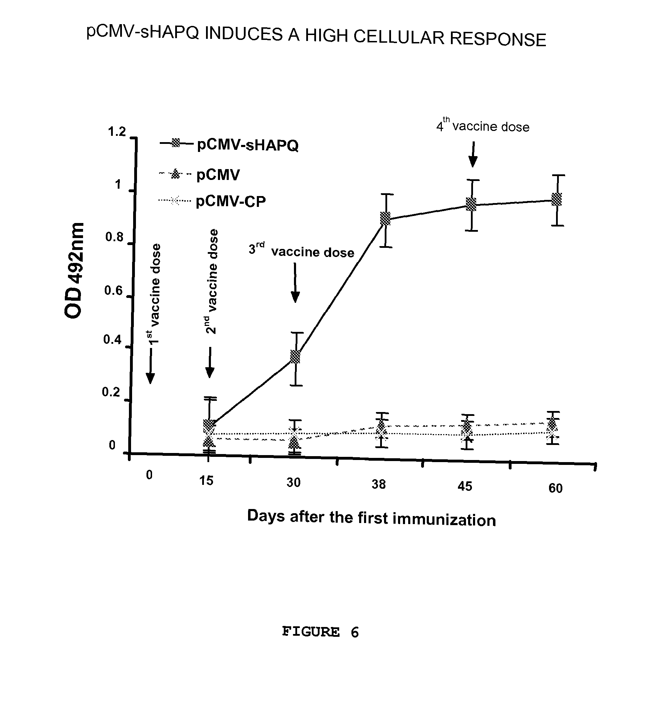

Figure 6 is a graph showing the results obtained when vaccinating pigs with different DNA vaccines (pCMV-CP, pCMV-sHAPQ and pCMV), a high induced antibody response against the p30 protein of ASFV being observed when plasmid pCMV-sHAPQ is used. The standard deviations within each group (8 pigs) are depicted.

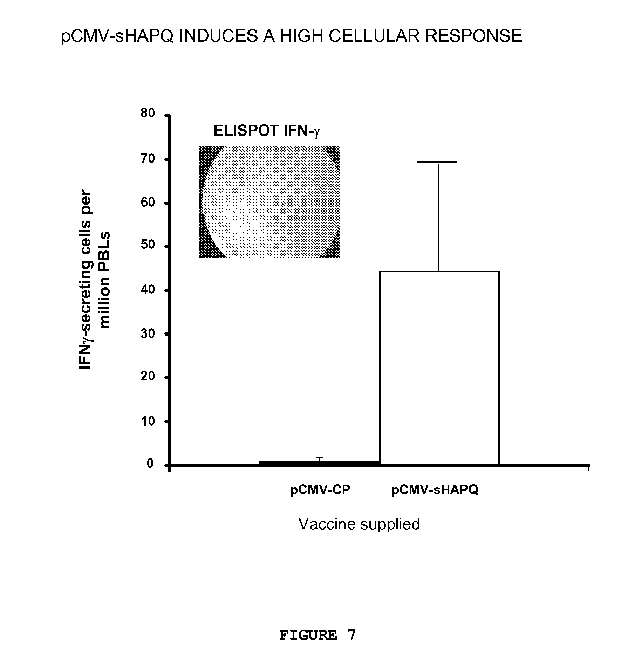

Figure 7 is a graph showing the results obtained when vaccinating pigs with different DNA vaccines (pCMV-CP and pCMV-sHAPQ), a better induced cellular response against the p30 protein of ASFV being observed when plasmid pCMV-sHAPQ is used. The standard deviations within each group (8 pigs) are depicted.

### Detailed Description of the Invention

In one aspect, the invention relates to a gene construct, hereinafter "gene construct of the invention", comprising:
a) a polynucleotide (A) selected from:
   (i) a polynucleotide comprising the nucleotide sequence encoding the hemagglutinin (HA) of African swine fever virus (ASFV);
   (ii) a functionally equivalent fragment of said polynucleotide defined in (i); and
   (iii) a functionally equivalent variant of said polynucleotide defined in (i) showing a minimum identity of 80% with respect to the encoding sequence of the HA of ASFV; and
b) a polynucleotide (B) comprising the nucleotide sequence encoding an antigen.

The polynucleotide (A) comprises the nucleotide sequence encoding the HA of ASFV, or a functionally equivalent fragment of said nucleotide sequence encoding the HA of ASFV, or a functionally equivalent variant of said nucleotide sequence encoding the HA of ASFV showing a minimum identity of 80% with respect to the encoding sequence of the HA of ASFV.

In a particular embodiment, the polynucleotide (A) comprises, or is formed by, the full length nucleotide sequence encoding the HA of ASFV.

The term "HA of ASFV" generally relates to a protein the amino acid sequence of which comprises or is formed by the amino acid sequence of the hemagglutinin (HA) of ASFV and includes any HA of any strain or isolate of ASFV, regardless of its origin; said protein (HA) is responsible for the adhesion of erythrocytes to infected cells (Ruiz-Gonzalvo F & Coll JM. Characterization of a soluble hemagglutinin induced in African swine fever virus-infected cells. Virology. 1993, 196(2):769-77; Ruiz-Gonzalvo F et al. Functional and immunological properties of the baculovirus-expressed hemagglutinin of African swine fever virus. Virology, 1996, 218:285-289).

The first two sequences of the HA of ASFV were published at almost the same time in 1994, corresponding with the sequences coming from (i) a Spanish isolate of ASFV isolated in Badajoz in 1971, adapted to Vero cells (Rodriguez JM et al. African swine fever virus encodes a CD2 homolog responsible for the adhesion of erythrocytes to infected cells. J Virol. 1993 Sep; 67(9):5312-20) and from (ii) a virulent African isolate (Borca MV et al. An African swine fever virus gene with similarity to the T-lymphocyte surface antigen CD2 mediates hemadsorption. Virology. 1994; 199(2):463-8). After this time, the HA of several hemadsorbing isolates (capable of binding to erythrocytes) have been sequenced. These sequences maintain a high degree of similarity and all of them are characterized by having a series of extracellular domains with considerable homology to the T-lymphocyte surface antigen CD2 (Borca et al., 1994, cited above; Rodriguez et al., 2004, cited above.). Therefore, the present invention includes the use of any HA or functionally equivalent fragment of the HA of ASFV regardless of its origin. In a particular embodiment, said HA of ASFV is the HA of the BA71 isolate of ASFV, the full-length amino acid sequence of which (SEQ ID NO: 1) can be found in NCBI, accession number L16864, together with the encoding nucleotide sequence of said HA of ASFV.

As used in the present invention, the term "ASFV" relates to the African Swine Fever Virus (ASFV) and includes any strain of ASFV, regardless of its origin, for example, Spanish strain España 75 (E75), a highly virulent strain, Spanish strain BA71, corresponding to a strain of ASFV isolated in Badajoz in 1971 and adapted to the monkey kidney-derived stable Vero (V) cell line; said strain (BA71) is apathogenic and is completely sequenced (accession number U18466) (Yanez RJ et al. Analysis of the complete nucleotide sequence of African swine fever virus. Virology. 1995 Apr 1; 208(1):249-78), etc.

In another particular embodiment, the polynucleotide (A) comprises, or is formed by, a functionally equivalent fragment of said polynucleotide comprising the nucleotide sequence encoding the HA of ASFV. As used herein, the expression "functionally equivalent fragment of said polynucleotide comprising the nucleotide sequence encoding the HA of ASFV" [or "functionally equivalent fragment of said polynucleotide defined in (i)", as indicated in the definition of the gene construct of the invention] relates to a fragment of a polynucleotide comprising, or formed by, a nucleotide sequence encoding a functionally equivalent fragment of the HA of ASFV.

Likewise, in the sense used in this description, the expression "functionally equivalent fragment of the HA of ASFV" relates to a part or portion of the HA of ASFV conserving the capacity to direct or carry an antigen to which it is bound to an APC or to a sequence which is obtained from said HA of ASFV by means of modifying a variable number of residues but also conserving the capacity to direct or carry an antigen to which it is bound to an APC. Said capacity to carry the antigen to which it is bound to an APC seems to be due to the existence of regions in the HA of ASFV having a high degree of homology to the binding domains of the lymphocyte surface antigen CD2 to its receptors in APCs. Therefore, in a particular embodiment, said functionally equivalent fragment contains the sequence comprised between amino acids 21 and 204 of the HA of ASFV, region of the HA of ASFV substantially homologous (i.e., having a high degree of identity) to the binding domain of CD2 to its receptors in the CPAs (Borca et al., 2004, cited above; Rodriguez et al., 2004, cited above). The capacity to carry the antigen to which said fragment is bound to an APC and to thus induce an immune response in animals can be determined by means of conventional methods such as the assays described in Example 1. In a particular embodiment, the functionally equivalent fragment of the HA of ASFV maintaining the capacity to carry said antigen to an APC is a peptide comprising or formed by the sequence comprised between amino acids 21 and 204 of the HA of ASFV, referred to in this description as "soluble fraction of the hemagglutinin" (sHA) of ASFV (SEQ ID NO: 2).

In another particular embodiment, the polynucleotide (A) comprises or is formed by a functionally equivalent variant of said polynucleotide comprising the nucleotide sequence encoding the HA of ASFV showing a minimum identity of 80% with respect to the encoding sequence of the HA of ASFV. Said functionally equivalent variant of said polynucleotide comprising the nucleotide sequence encoding the HA of ASFV showing a minimum identity of 80% with respect to the encoding sequence of the HA of ASFV generally encodes a variant of the HA of ASFV which is functionally equivalent to the HA of ASFV.

In another particular embodiment, the polynucleotide (A) comprises or is formed by a functionally equivalent variant of said polynucleotide comprising the nucleotide sequence encoding the HA of ASFV showing at least an 80%, 82%, 84%, 86%, 88%, 90%, 92%, 94%, 96% or 98% identity at the level of the nucleotide sequence encoding the HA of ASFV.

Polynucleotide (B) comprises the nucleotide sequence encoding an antigen. As used herein, the term "antigen" relates to a peptide or protein molecule capable of binding to an antibody or to a T cell receptor when it is presented by molecules of the major histocompatibility complex (MHC). Therefore, said term includes any peptide or protein substance capable of being recognized by the immune system of a subject and/or capable of inducing in a subject a humoral immune response or a cellular immune response leading to the activation of B- and/or T-lymphocytes when it is introduced in a subject, which may occasionally require the antigen to contain or to bind to an epitope of Th cells. An antigen can have one or more epitopes (B and T epitopes).

By way of non-limiting illustration, said antigen can be:
(a) a peptide or protein capable of (suitable or designed for) inducing an immune response against an infectious disease;
(b) a peptide or protein capable of (suitable or designed for) inducing an immune response in animals, for example, human beings and domestic animals, including farm animals and pets;
(c) a peptide or protein capable of (suitable or designed for) inducing an immune response against a cancer cell;
(d) a peptide or protein capable of (suitable or designed for) inducing an immune response against an allergen;
(e) a peptide or protein capable of (suitable or designed for) inducing an improved response against an autoantigen; or
(f) a fragment (e.g., a domain) of any of said peptides or proteins (a) - (e) .

As used in this description, the term "peptide" or "protein" includes post-translational modifications of the peptide or protein, for example, glycosylations, acetylations, phosphorylations and the like.

In a particular embodiment, said antigen is a peptide or protein capable of (suitable or designed for) inducing an immune response against an infectious disease, such as an infectious disease in animals caused by pathogenic microorganisms of animals, including human beings, for example, viruses, bacteria, fungi, infectious parasites, prions, etc., relevant in human or animal health. The antigens expressed by the gene construct of the invention are recombinant products identical or similar to the natural antigens of a microorganism, and capable of inducing a specific immune response for that microorganism.

Illustrative, non-limiting examples of infectious viruses found in animals include viruses from the following families: *Arteriviridae, Retroviridae, Picornaviridae, Calciviridae, Togaviridae, Flaviridae, Coronoviridae, Rhabdoviradae, Filoviridae, Paramyxoviridae, Orthomyxoviridae, Bungaviridae, Arenaviridae, Reoviridae, Birnaviridae, Hepadnaviridae, Parvoviridae* (parvovirus), *Papovaviridae, Adenoviridae, Herpesviridae, Poxviridae, Iridoviridae*, etc. Said infectious viruses include pathogens of pigs, for example, African swine fever virus (ASFV), classical swine fever virus (CSFV), porcine parvovirus (PPV), porcine reproductive and respiratory syndrome virus (PRRSV), Aujeszky's disease virus (ADV), foot-and-mouth disease virus (FMDV), transmissible gastroenteritis virus (TGEV), porcine circovirus, etc., cattle pathogens, such as bovine viral diarrhea virus (BVDV), infectious bovine rhinotracheitis virus (IBRV), blue tongue virus (BTV), etc., rabbit pathogens, such as rabbit hemorrhagic disease virus (RHDV), etc., bird pathogens, for example, infectious bursal disease virus (IBDV) or Gumboro disease, avian pneumovirus, etc.

Illustrative, non-limiting examples of bacteria include both Gram-positive bacteria, e.g., *Pasteurella sp*., *Staphylococcus sp., Streptococcus sp*., etc., and Gram-negative bacteria, e.g., *Escherichia coli, Pseudomonas sp., Salmonella sp*., etc. Specific examples of infectious bacteria include: *Helicobacter pylori, Borrelia burgdorferi, Legionella pneumoplailia, Mycobacteria sp*. (e.g., *M. tuberculosis, M. avium, M. intracellulare, M. kansaii, M. gordonae), Staphylococcus aureus, Neisseria gonorrhoeae, Neisseria meningitidis, Listeria monocytogenes, Streptococcus pyogefaes* (Group A Streptococcus), *Streptococcus agalactiae* (Group B Streptococcus), Streptococcus (viridans group), *Streptococcus faecalis, Streptococcus bovis, Streptococcus* (anaerobic sp.), *Streptococcus pneumoniae, Campylobacter sp., Enterococcus sp.,* ***Haemophilus influenzae, Bacillus aratracis,*** *Corynebacteriumdiphtlzeriae, Corynebacterium sp., Erysipelothrix rhusiopathiae, Clostridium perfringers, Clostridium tetani, Enterobacter aerogenes, Klebsiella pyzeunaoniae, Pasturella multocida, Bacteroides sp*., *Fusobacterium nucleatum, Streptobacillus moniliformis, Treponemapallidium, Treponema pertenue, Leptospira, Rickettsia, Actinornyces israelli, Chlamydia*, etc.

Illustrative, non-limiting examples of infectious fungi include *Cryptococcus neoformans, Histoplasma capsulatum, Coccidioides immitis, Blastomyces dermatitidis, Chlamydia trachomatis* and *Candida albicans.*

By way of non-limiting illustration, infectious parasites include protozoa, such as *Plasmodium sp*., agents causing malaria, e.g. *P. falciparum, P. malariae, P. ovale, P. vivax, etc., Leishmania sp*., agents causing leishmaniasis, e.g., *L. major, L. donovani, L. infantum, L. braziliensis, L. panamensis, L. mexicana,* etc., *Toxoplasma gondii, Schistosoma* sp., etc., as well as parasitic nematodes, such as *Dirofilaria immitis*, etc.

Prions or prion proteins are acellular, pathogen and transmissible particles causing diseases affecting the central nervous system (CNS), which include transmissible spongiform encephalopathies. Illustrative, non-limiting examples of prion diseases in human beings include classic Creutzfeldt-Jakob disease (CJD), the variant of Creutzfeldt-Jakob disease (CJD), Gerstmann-Straussler-Scheinker disease, fatal familial insomnia, etc. Illustrative, non-limiting examples of prion diseases in other animals are bovine spongiform encephalopathy, sheep scrapie, transmissible mink encephalopathy, feline spongiform encephalopathy, ungulate spongiform encephalopathy, chronic wasting diseases (mules, deer, elk, etc.), etc.

In another particular embodiment, said antigen is a peptide or protein capable of (suitable or designed for) inducing an immune response in animals, for example, human beings and domestic animals, including farm animals and pets.

In another particular embodiment, said antigen is a peptide or protein associated to a tumor or to a cancer ("tumor marker") capable of (suitable or designed for) inducing an immune response against a tumor or cancer cell, therefore the gene construct of the invention can be used in the treatment of cancers by means of stimulating a specific immune response of an antigen against a tumor antigen. Illustrative, non-limiting examples of cancers that could be potentially treated according to the teachings of the present invention include bile duct cancer, brain cancer, breast cancer, cervical cancer, choriocarcinoma, colon cancer, endometrial cancer, esophageal cancer, stomach cancer, intraepithelial neoplasms, lymphomas, liver cancer, lung cancer (e.g., small cell and non-small cell lung cancer), melanoma, neuroblastomas, mouth cancer, ovarian cancer, pancreatic cancer, prostate cancer, rectal cancer, sarcomas, skin cancer, testicular cancer, thyroid cancer, and renal cancer, as well as other carcinomas and sarcomas.

The tumor antigens or antigenic determinants for the treatment of cancers can be selected by a person skilled in the art in view of the state of the art [Renkvist et al., Cancer Immunol. Immunother. 50:3-15 (2001)], said antigens and antigenic determinants being included within the scope of the present invention. Representative examples of said antigens or antigenic determinants include: Her2 (breast cancer); GD2 (neuroblastoma); EGF-R (malignant glioblastoma); CEA (medullary thyroid cancer); CD52 (leukemia); human melanoma gp100 protein; human melanoma melan-A/MART-1 protein; tyrosinase; NA17-A protein; MAGE-3 protein; p53 protein; HPV16E7 protein; and antigenic fragments of said peptides or proteins.

In another particular embodiment, said antigen is a peptide or protein capable of (suitable or designed for) inducing an immune response against an allergen.

As used in this description, the term "allergen" relates to a peptide or protein to which a subject is sensitive and causes an immune reaction, for example, allergen extracts of pollens, allergen extracts of insects, allergen extracts of foods or foodstuffs, components present in saliva, pincers or stingers of insects inducing a sensitivity reaction in a subject, components present in plants inducing a sensitivity reaction in a subject, etc. Illustrative, non-limiting examples of allergens include protein extracts of pollens, e.g., of *Lolium perenne, Poa pratense, Phleum pratense, Cynodon dactylon, Festuca pratensis, Dactylis glomerata, Secale cereale, Hordeum vulgare, Avena sativa, Triticum sativa, Artemisia vulgaris, Chenopodium album, Plantago lanceolata, Taraxacum vulgare, Parietaria judaica, Salsola kali, Urtica dioica, Olea europea, Platanus sp*., *Cupressus sp*., etc.; protein extracts of insects, e.g., of *Dermatophagoides pteronyssinus, Dermatophagoides farinae, Acarus siro, Blomia tropicalis, Euroglyphus maynei, Glyciphagus domesticus, Lepidoglyphus destructor, Tyrophagus putrescentiae,* etc.; protein extracts of fungi or of animal dander, e.g., *Penicillium sp., Alternaria alternata, Cladosporium herbarum,* dog dander, cat dander, horse dander, etc.; protein extracts of foods or foodstuffs, etc.

In another particular embodiment, said antigen is a peptide or protein capable of (suitable or designed for) inducing an improved response against an autoantigen. As used herein, the term "autoantigen" relates to peptides or proteins encoded by the DNA of the subject and products generated by proteins or RNA encoded by the DNA of the subject. Examples of autoantigens are described in WO 02/56905.

In another particular embodiment, said antigen is a fragment of any of said previously defined peptides or proteins (a)-(e); said fragment will generally include an antigenic domain of said peptides or proteins.

In another particular embodiment, said antigen is a fusion protein comprising the p54 and p30 proteins of ASFV.

The gene construct of the invention comprises a polynucleotide (A) and a polynucleotide (B). Said polynucleotides (A) and (B) can bind in any order. Therefore, in a particular embodiment, the 3' end of said polynucleotide (A) is bound to the 5' end of said polynucleotide (B), whereas in another particular embodiment, the 5' end of said polynucleotide (A) is bound to the 3' end of said polynucleotide (B). Both polynucleotides (A) and (B) can bind directly or through a polynucleotide (C), encoding a spacer peptide, between said polynucleotides (A) and (B). Examples of spacer peptides could range from marker peptides against which there is a specific antibody which enables tracking the expression of the fusion (e.g.: c-myc) to any peptide which is artificially generated as a result of the fusion of the specific sequences of the HA and of the specific antigen.

In a particular embodiment, it would be possible for the polynucleotide (B) to comprise the encoding nucleotide sequences of two or more antigens (e.g., arranged in tandem) for the purpose of immunizing the animal against different pathogens in one and the same immunization; and theoretically, even for the polynucleotide (A) to be bound by the 5' end to a polynucleotide (B) and by the 3' end to another polynucleotide (B).

In a particular embodiment, the gene construct of the invention comprises the nucleotide sequence defined in SEQ ID NO: 5.

The gene construct of the invention can be obtained by means of the use of techniques widely known in the state of the art [Sambrook et al., "Molecular cloning, a Laboratory Manual", 2nd ed., Cold Spring Harbor Laboratory Press, N.Y., 1989] and, if desired, it can be inserted in a suitable vector.

Therefore, in another particular embodiment, the invention relates to a vector, hereinafter "vector of the invention", comprising a gene construct of the invention. Said vector can be a storage, multiplication or expression vector, typically an expression vector, and if desired, it can be used to transform cells or organisms susceptible of being transformed by said vector. Said cells can be prokaryotic or eukaryotic. In a particular embodiment, the vector of the invention is a vector useful for transforming eukaryotic cells, e.g., animal cells, advantageously mammalian cells.

In a particular embodiment, the vector of the invention comprises a gene construct of the invention operatively bound to a regulatory sequence of the expression of said polynucleotides (A) and (B) [and, where appropriate, (C)] thus forming an expression cassette. As used in this description, the expression "operatively bound" means that the protein encoded by said polynucleotides (A) and (B) [and, where appropriate, (C)], is expressed in the correct reading frame under the control of the control or regulatory sequences of expression. The control or regulatory sequences of expression are sequences controlling and regulating transcription and, where appropriate, translation of a protein, and include promoter sequences, sequences encoding transcriptional regulators, ribosome binding sequences (RBS) and/or transcription terminator sequences. However, in a particular embodiment said control sequence of expression is functional in prokaryotic cells and organisms, for example, bacteria, etc., advantageously in another particular embodiment, said expression control sequence is functional in eukaryotic cells and organisms, for example mammalian cells, mammalian cell lines, etc.

Additionally, if desired, the vector of the invention further comprises a marker or gene encoding a motif or phenotype which allows selecting and/or locating the cells or organisms transformed with said vector.

In a particular embodiment, the vector of the invention is a plasmid which, when introduced in a host cell, is integrated (or not) into the genome of said cell. Illustrative, non-limiting examples of vectors of those in which the gene construct of the invention can be inserted include the vector pCMV marketed by Clontech.

The vector of the invention can be obtained by conventional methods known by persons skilled in the art [Sambrook et al., "Molecular cloning, a Laboratory Manual", 2nd ed., Cold Spring Harbor Laboratory Press, N.Y., 1989 Vol. 1-3].

In another aspect, the invention relates to a eukaryotic or prokaryotic cell comprising the gene construct of the invention or the vector of the invention. In a particular embodiment, said cell is a prokaryotic cell, e.g., a bacterial cell; in another particular embodiment, said cell is a eukaryotic cell, such as an animal cell, e.g., a mammalian cell.

In another aspect, the invention relates to a fusion protein, hereinafter "fusion protein of the invention", encoded by the encoding sequence present in the gene construct of the invention. Therefore, said fusion protein of the invention comprises:
a) a polypeptide (A') selected from:
   (i) a polypeptide comprising the amino acid sequence of the hemagglutinin (HA) of African swine fever virus (ASFV);
   (ii) a functionally equivalent fragment of said polypeptide defined in (i); and
   (iii) a functionally equivalent variant of said polypeptide defined in (i) showing a minimum identity of 80% with respect to the amino acid sequence of the HA of ASFV; and
b) a polypeptide (B') comprising the amino acid sequence of an antigen.

In a particular embodiment, said polypeptide (A') comprises, or is formed by, the complete amino acid sequence of the HA of ASFV; in a specific embodiment, said polypeptide (A') comprises, or is formed by, the amino acid sequence shown in SEQ ID NO: 1 corresponding to the amino acid sequence of the HA of the BA71 isolate of ASFV.

In another particular embodiment, said polypeptide (A') comprises, or is formed by, a functionally equivalent fragment of the HA of ASFV, such as a region of the HA of ASFV comprising the region homologous to the binding domains of CD2 to APC; in a specific embodiment, said polypeptide (A') comprises, or is formed by, the amino acid sequence shown in SEQ ID NO: 3 corresponding to the amino acid sequence of the sHA fraction of ASFV.

In another particular embodiment, said polypeptide (A') comprises, or is formed by, a functionally equivalent variant of said polypeptide comprising the amino acid sequence of the HA of ASFV showing a minimum identity of 80% with respect to the amino acid sequence of the HA of ASFV; in a specific embodiment, said polypeptide (A') comprises a functionally equivalent variant of the HA of ASFV showing at least an 80%, 82%, 84%, 86%, 88%, 90%, 920, 940, 96% or 98% identity at the level of the amino acid sequence of the HA of ASFV.

The polypeptide (B') comprises, or is formed by, the amino acid sequence of an antigen; specifically by the antigen encoded by the polynucleotide (B) present in the gene construct of the invention. Therefore, in a particular embodiment, said polypeptide (B') comprises, or is formed by, the amino acid sequence of an antigen such as any of those previously defined in relation to the polynucleotide (B).

In a particular embodiment, the polypeptide (B') is a fusion protein comprising the p54 and p30 proteins of ASFV. In another particular embodiment, the polypeptide of the invention has the sequence SEQ ID NO: 4.

Said polypeptides (A') and (B') can bind in any order. Therefore, in a particular embodiment, the carboxyl-terminal end of said polypeptide (A') is bound to the amino-terminal end of said polypeptide (B'), whereas in another particular embodiment, the amino-terminal end of said polypeptide (A') is bound to the carboxyl-terminal end of said polypeptide (B'). Both polypeptides (A') and (B') can bind directly or through a polypeptide (C'), a spacer peptide, between said polypeptides (A') and (B').

The fusion protein of the invention can be used for therapeutic purposes, e.g., in the preparation of immunotherapeutic compositions and vaccines.

In another aspect, the invention relates to a host cell, hereinafter "host cell of the invention", containing the gene construct of the invention, or the vector of the invention, or the fusion protein of the invention. Said host cell of the invention is capable of expressing the protein of the invention and can be obtained by means of transformation, transfection or infection with a vector of the invention, as previously mentioned, by conventional methods known by persons skilled in the art [Sambrok et al., 1989]. Suitable host cells include prokaryotic cells, yeasts or eukaryotic cells, more preferably, an antigen-presenting cell (APC). Different expression systems can be used to express the fusion protein of the invention, for example, expression systems based on the culture of insect cells, mammalian cells, etc. The baculovirus systems for producing proteins in insect cells are well known in the state of the art. Likewise, some examples of suitable host mammalian cell lines include but are not limited to NS/0 cells, L cells, C127, 3T3, Chinese hamster ovarian (CHO) cells, human embryonic kidney cells (HEK-293), HeLa and BHK; CV-1 cells (ATCC CCL70) and COS-7 cells, both derived from monkey kidney, etc. The host cell is preferably an antigen-presenting cell (APC), particularly a dendritic cell, a macrophage or a B cell, and among the dendritic cells, preferably Langerhans cells or follicular dendritic cells, bone marrow cells or blood cells.

In another aspect, the invention relates to a method for producing the fusion protein of the invention. In a particular embodiment, the fusion protein of the invention can be obtained by conventional methods known by persons skilled in the art, for example, by means of the expression of the nucleotide sequence of the gene construct of the invention encoding said fusion protein in suitable host cells. Therefore, the fusion protein of the invention can be obtained by means of a process comprising culturing a host cell of the invention, comprising the gene construct of the invention or the vector of the invention, under suitable conditions for the expression of said protein. If desired, the fusion protein of the invention thus obtained can be isolated, and optionally purified, by conventional methods.

The fusion protein of the invention can alternatively be obtained by conventional methods of chemical synthesis of proteins known by persons skilled in the art. In a particular embodiment, it may be suitable or desirable for said fusion protein to include a peptide sequence which aids in the isolation or purification thereof (e.g., a histidine tail, an epitope against which there are antibodies (e.g., c-myc), etc.).

In another aspect, the invention relates to a pharmaceutical composition, hereinafter "pharmaceutical composition of the invention", comprising a therapeutically effective amount of a gene construct of the invention, or of a vector of the invention, or of a fusion protein of the invention, or of a host cell of the invention, together with a pharmaceutically acceptable carrier.

The pharmaceutical composition of the invention will be presented in a suitable pharmaceutical dosage form for its administration to a subject. To that end, the pharmaceutical composition of the invention will include pharmaceutically acceptable carriers and excipients necessary for preparing the chosen pharmaceutical dosage form. The pharmaceutical dosage form of the active component (gene construct of the invention, vector of the invention, fusion protein of the invention or host cell of the invention) present in the pharmaceutical composition of the invention may vary within a broad range of possibilities known by persons skilled in the art, depending, on other factors, on the nature of said active component (nucleic acid, protein or cell) and on the chosen route of administration. In this sense, the pharmaceutical composition of the invention can be administered to a subject by any suitable route of administration, e.g., orally, parenterally, etc.

In a particular embodiment, the pharmaceutical composition of the invention comprises a gene construct of the invention or a vector of the invention together with a pharmaceutically acceptable carrier. In this case, said gene construct or vector of the invention can be administered in the form of naked DNA (i.e., simply diluted in a physiological solution), administered in any of its possible forms and using the most diverse routes of inoculation (reviewed on webpage: www.dnavaccine.com). Generally, for its administration to a subject, the gene construct of the invention will be included in a vector of the invention suitable for its administration to a subject. By way of non-limiting illustration, said vector of the invention can be a viral vector, for example, a vector based on a retrovirus, or on an adenovirus, etc., or a nonviral vector, e.g., a DNA-liposome complex, a DNA-polymer complex, a DNA-polymer-liposome complex, etc. [see "Nonviral Vectors for Gene Therapy", edited by Huang, Hung and Wagner, Academic Press (1999)]. Said vectors can be administered directly to the subject by conventional methods. Said vectors can alternatively be used to transform, transfect or infect cells, for example mammalian cells, *ex vivo,* and, to subsequently implant them in the animal body to obtain the desired therapeutic effect. Said cells will be formulated in a suitable medium which does not adversely affect the viability of said cells for their administration to the subject receptor.

In another particular embodiment, the pharmaceutical composition of the invention comprises a fusion protein of the invention together with a pharmaceutically acceptable carrier. In this case, by way of non-limiting illustration, the pharmaceutical composition of the invention can be formulated in a solid (e.g., tablets, capsules, sugar-coated tablets, granules, suppositories, etc.) or liquid (e.g., solutions, suspensions, emulsions, etc.) pharmaceutical dosage form for its oral, parenteral (e.g., intramuscular, subcutaneous, intravenous, etc.) administration, etc. In each case the suitable pharmaceutically acceptable carriers and excipients will be chosen for the pharmaceutical dosage form and route of administration chosen. Information on said carriers and excipients and on said pharmaceutical dosage forms of the fusion protein of the invention can be found in Galenic pharmacy treaties. A review of the different pharmaceutical dosage forms of drugs in general and of the processes for preparing them can be found in the book "Tratado de Farmacia Galénica", by C. Faulí i Trillo, 1st Edition, 1993, Luzán 5, S.A. de Ediciones.

In another particular embodiment, the pharmaceutical composition of the invention comprises a host cell of the invention together with a pharmaceutically acceptable carrier. The host cells of the invention can be administered to the subject so that they express the fusion protein directly on the actual subject.

The pharmaceutical composition of the invention comprises at least one active component, such as a gene construct of the invention, a vector of the invention, a host cell of the invention or a fusion protein of the invention, in a therapeutically effective amount. As used herein, the expression "therapeutically effective amount" relates to the amount of active component calculated to produce the desired effect, and it is generally determined, among others, by the typical characteristics of the active component and the therapeutic effect to be achieved. Therefore, the dose of active component to be administered to a subject will be a therapeutically effective amount and may vary within a broad range. The pharmaceutical composition of the invention can be administered one or more times a day for preventive or therapeutic purposes. The dose of active component to be administered will depend on a number of factors, including the characteristics of the product to be administered, such as, for example, its biological half-life and activity, the concentration of the product in the pharmaceutical composition, the chosen pharmaceutical dosage form, etc. For this reason, the doses mentioned in this invention must be considered only as a guideline for the person skilled in the art, and this person must adjust the doses according to the previously mentioned variables. By way of a merely non-limiting illustration, in a particular embodiment the pharmaceutical composition of the invention can be administered one or more times a day in a typical amount comprised between 100 µg and 1000 µg of vector/subject, preferably, between 200 µg and 800 µg of vector/subject, although variations may arise depending on the response of the individual subject to said pharmaceutical composition, as well as on the type of the chosen pharmaceutical formulation and on the period of time and interval in which such administration is performed. The dose of the pharmaceutical composition of the invention can be repeated, depending on the condition of the subject and his or her evolution, at time intervals (days, weeks or months) which will have to be established in each case by the specialist. By way of non-limiting illustration, the pharmaceutical composition of this invention will generally be administered in from one up to four vaccine doses every day.

As used in this description, the term "subject" includes any animal having an immune system, preferably mammals, for example, swine (e.g., pigs, etc.).

Although, in principle, the pharmaceutical composition of the invention can be used to treat any subject, in a particular embodiment, the pharmaceutical composition of the invention is especially useful for treating swine (e.g., pigs, etc.).

In another aspect, the invention relates to the use of a gene construct of the invention, or of a vector of the invention, or of a fusion protein of the invention or of a host cell of the invention, in the preparation of a pharmaceutical composition for the treatment or prevention of a disease caused by an organism expressing or containing said antigen. In a particular embodiment, said gene construct of the invention, vector of the invention, host cell of the invention or fusion protein of the invention, can be used in the preparation of a pharmaceutical composition for the treatment or prevention in swine of a disease caused by a porcine pathogen expressing or containing said antigen. Illustrative examples of porcine pathogens have previously been included.

The fusion protein of the invention can be used to produce, by means of conventional methods known by persons skilled in the art, antibodies recognizing said fusion protein of the invention. Said antibodies, or fragments thereof with capacity to bind to said fusion protein of the invention, can be useful for the prevention and/or treatment of a disease caused by an organism, e.g., a pathogenic organism, containing the antigen present in the fusion protein of the invention. Said antibodies, or fragments thereof with capacity to bind to said fusion protein of the invention, hereinafter antibody of the invention, are an additional aspect of this invention. In a particular embodiment, said antibody recognizes a specific epitope of an antigen as has been previously mentioned.

As used in this description, the term "antibody" intends to include both chimeric or recombinant antibodies and monoclonal antibodies and polyclonal antibodies or proteolytic fragments thereof, such as Fab or F(ab')2 fragments, etc. Furthermore, the DNA encoding the variable region of the antibody can be inserted in other antibodies to thus produce chimeric antibodies. Single-chain antibodies (scFv) can be polypeptides formed by single chains having the typical capacity of an antigen-binding antibody and comprising a pair of amino acid sequences homologous or analogous to the variable regions of light and heavy chains of immunoglobulins (VH-VL or scFv binding). The polypeptides analogous to the variable regions of the light and heavy chains of an antibody can bind, if desired, through a binding polypeptide. Methods for producing antibodies are widely known by a person skilled in the art and are included in the state of the art.

In another aspect, the invention relates to a pharmaceutical composition comprising an antibody of the invention. Likewise, in another aspect, the invention relates to the use of an antibody of the invention, recognizing a fusion protein of the invention, in the preparation of a pharmaceutical composition for the prevention and/or treatment of a disease caused by an organism containing said antigen present in said fusion protein.

The fusion protein of the invention can furthermore be used to produce cells of the immune system, such as B cells, T cells, dendritic cells, etc., recognizing said protein. Said isolated cells with the capacity to recognize said fusion protein of the invention, hereinafter cells of the immune system of the invention, can be useful for the prevention and/or treatment of a disease caused by a pathogenic organism containing the antigen present in the fusion protein of the invention and are an additional aspect of this invention.

In another aspect, the invention relates to a pharmaceutical composition comprising a cell of the immune system of the invention. Likewise, in another aspect, the invention relates to the use of a cell of the immune system of the invention, such as a B cell, a T cell, a dendritic cell, etc., recognizing a fusion protein of the invention, in the preparation of a pharmaceutical composition for the prevention and/or treatment of a disease caused by an organism containing said antigen present in said fusion protein.

In another aspect, the invention relates to a method for enhancing the immune response against an antigen comprising administrating to a subject a therapeutically effective amount of a gene construct of the invention in which said polynucleotide (B) comprises the nucleotide sequence encoding said antigen, or of a vector of the invention comprising said gene construct, or of a fusion protein of the invention comprising said antigen or of a host cell expressing the antigen. The administration of said gene construct of the invention, or of said vector of the invention or of said fusion protein of the invention will be carried out in the form of a pharmaceutical composition, the characteristics of which have already been mentioned (pharmaceutical composition of the invention).

As shown in Example 1 accompanying the present description, the experiments conducted by the inventors clearly show that, after the immunization of pigs with a gene construct encoding a fusion protein comprising the sHA fraction of ASFV and a specific antigen, there is high immune response against said antigen, which is much greater than that observed in control animals. Thus, the HA of ASFV, or a functionally equivalent fragment thereof, such as the sHA fraction of ASFV, with the capacity to carry the antigen to which it is bound to an APC, has an adjuvant effect in DNA vaccination when it is administered together with an antigen, an effect enhancing or stimulating the immune response in the subject after its administration to said subject occurring.

Therefore, in another aspect, the invention relates to the use of the HA of ASFV or of a functionally equivalent fragment thereof or of a functionally equivalent variant thereof showing a minimum identity of 80% with the HA of ASFV as an adjuvant in the preparation of a pharmaceutical composition, such as an immunotherapeutic composition or vaccine, comprising an antigen.

Likewise, in another aspect, the invention relates to the use of the HA of ASFV or a functionally equivalent fragment thereof or of a functionally equivalent variant thereof showing a minimum identity of 80% with the HA of ASFV in the preparation of a composition for enhancing the immune response against an antigen jointly administered with said immune response-enhancing composition.

The following example illustrates the invention and must not be considered as limiting the scope thereof.

### EXAMPLE 1

### Construction of plasmids pCMV-CP and pCMV-sHAPQ and immunization of pigs with said plasmids

### Construction of plasmids pCMV-CP and pCMV-sHAPQ

The way to obtain a fusion protein, generically identified as Chimeric Protein (CP), comprising the amino acid sequence of the p30 protein and of the p54 protein of African swine fever virus (ASFV) (SEQ ID NO: 3), is described. The sequence corresponding to the p30 protein of ASFV is included between amino acids 138 and 139 of the p54 protein and comprises from residue 139 to 341 of the chimeric protein, CP maintaining both the antigenic and immunogenic properties of both proteins of ASFV (Barderas MG, Rodriguez F, Gomez-Puertas P, Aviles M, Beitia F, Alonso C, Escribano JM. Antigenic and immunogenic properties of a chimera of two immunodominant African swine fever virus proteins. Arch Virol. 2001; 146(9):1681-91). As a result of the fusion of the sHA with CP, amino acid 138 of the p54 of ASFV is substituted, replacing the proline with a threonine (Thr 138 in SEQ ID NO: 3).

Briefly, the DNA sequence encoding said CP fusion protein (Barderas et al., 2001, cited above) was cloned into plasmid pCMV (Clontech) under the control of the human cytomegalovirus (CMV) immediate-early promoter to generate plasmid pCMV-CP, as shown in Figure 4. Said plasmid pCMV-CP is capable of expressing said CP fusion protein, resulting from the fusion of the p30 and p54 proteins of ASFV.

After the digestion of the baculovirus transfer plasmid pBacpakp54/p30 (Barderas et al., 2001, cited above) with the restriction enzyme BamHI, the DNA fragment corresponding to the open reading frame encoding the CP fusion protein, flanked by compatible sticky ends for the ligation thereof in plasmid pCMVLII (modified by Rodriguez et al. Journal of Virology, 2001, 75:10421-10430), as well as in plasmid pCMV-sHA (described below), was obtained.

In addition, the DNA fragment encoding said CP was cloned into said vector (pCMV) as a fusion molecule with the DNA sequence encoding the soluble fraction of the hemagglutinin (sHA) of ASFV. As a result of the fusion of the sHA with CP two additional amino acids are introduced: arginine 185 and serine 186 (Arg 185 and Ser 136 in SEQ ID NO: 4), generating plasmid pCMV-sHAPQ (Figure 5). Said plasmid is capable of expressing said CP protein (formed by the p30 and p54 proteins of ASFV) as a fusion protein with the sHA fraction (identified as sHA-CP in Figure 5) (SEQ ID NO: 4).

Briefly, the DNA fragment encoding the sHA fraction of ASFV was obtained by means of the polymerase chain reaction using primers:
Up HA Not I 5'-GCGGCCGCCATGTGGAGTACTTTAAATCAAAC-3'
Down HA EagI 5'-CGGCCGAGATCTTGTGGATAAATAATTTTG-3',
and the DNA of the BA71 isolate of ASFV as a template of the reaction. The PCR reaction consisted of 30 reaction cycles, each consisting of 30 seconds of denaturing at 95°C, followed by 1 minute of annealing at 55°C and 2 minutes of extension at 72°C.

The amplification product corresponding to the ORF of the sHA fraction of ASFV was subcloned into the pGEMT-easy vector (Promega) and, after its digestion with restriction enzymes EagI and NotI, allowed its purification and cloning into the only NotI site of pCMV (Clontech) to obtain the pCMV-sHA vector.

Finally, the DNA encoding the CP, product of the digestion of pBacpackp54/p30 (see above), was cloned as a fusion molecule to the DNA encoding the fragment fused to the sHA fraction after the digestion of plasmid pCMV-sHA with BglII, to obtain the final plasmid pCMV-sHAPQ (Figure 5), comprising the nucleotide sequence shown in SEQ ID NO: 5, which is capable of expressing fusion protein sHA-CP.

### Inoculation of animals with the pCMV-CP and pCMV-sHAPQ constructs

Three groups of Large white/landrace pigs (8 pigs per group) were intramuscularly inoculated with each of the following plasmids: pCMV (control plasmid), pCMV-CP and pCMV-sHAPQ. Up to 4 vaccine doses distributed over 15 days were supplied, inoculating 600 µg (1.5 ml of physiological saline solution) of plasmid per pig (distributed among the muscles of the neck and of the hindquarters and subcutaneously in the ear).

The animals were bled before each vaccine dose and 6 weeks after the last administered dose (at which time the memory response had already been established), in order to be able to measure, from the serum obtained, the antibodies specifically directed against the antigens of ASFV. Said measurement can be made by conventional techniques, such as by means of an ELISA or by means of a Western Blot. The results shown correspond to an assay in which the recombinant p30 protein of ASFV expressed in baculovirus was used as an antigen to cover the ELISA plates (Figure 6) as has been previously described (Oviedo et al. 1997, J Virol Methods 64:27-35) . As can be observed, the immune response when the construct (pCMV-sHAPQ) encoding fusion protein sHA-CP is injected, is much higher than that occurring when a plasmid encoding the CP fusion protein without the sHA fraction (pCMV-CP) or with the control plasmid (pCMV) is injected.

To study the induction of the cellular response (T-CD4⁺ cells and T-CD8⁺ cells), the cellular response was measured in all the pigs immunized with the control plasmid (pCMV) and with plasmids pCMV-CP and pCMV-sHAPQ, 6 weeks after the last vaccine dose was administered (to measure the memory T response), using to that end the ELISPOT technique specific for interferon gamma (IFN-γ). This methodology allows quantifying the number of T cells which specifically secrete IFN-γ upon recognizing a specific antigen (a peptide, a protein, complete virus ...), after the vaccination with the different plasmids. Thus, the white cells (PBLs) were obtained from a complete blood sample independently of the erythrocytes, to then be cultured *in vitro* in culture plates (Millipore) pre-coated with an anti-IFN-γ antibody (Pharmingen), according to conventional protocols (Immunological Methods 2006). The cells were grown for 18 hours in the presence of the recombinant p30 protein of ASFV (Figure 7) or in the presence of an irrelevant protein (albumin), negative control. Finally, the cells were removed and the plates were developed as if it were a normal ELISA, using specific reagents from the company Pharmingen. Each of the dots observed after the developing (see insert in Figure 7) corresponds to a specific cell for the specific stimulus.

### Improvement of the induced immune response after DNA vaccination

A specific response of antibodies against the p30 protein of ASFV detectable by ELISA was not detected in any of the pigs immunized with pCMV-CP (plasmid expressing the fused p30 and p54 proteins, i.e., CP) (Figure 6). As occurs for the antibody response, no animal immunized with pCMV-CP induces cellular response (Figure 7). Despite the fact that this same construct is immunogenic in mice (laboratory data not published), it is not functional in pigs.

As a summary, the results obtained allow making the following comments:
A) all the animals immunized with pCMV-sHAPQ developed a high response of antibodies specific against the antigenic p30 protein of ASFV (Figure 6); therefore, the induced antibody response after vaccination is enhanced by directing the antigens of ASFV to antigen-presenting cells (APC) by means of their fusion to the sHA;
B) the pCMV-sHAPQ-induced antibody response reaches a plateau after the 3^{rd} vaccination dose, whereby additional vaccine doses do not seem to have any additional effect (Figure 6); and
C) all the pigs vaccinated with pCMV-sHAPQ developed a cellular response; all the animals immunized with pCMV-sHAPQ developed a high IFN-γ response after the stimulation with p30 of ASFV (Figure 7); therefore, the induced cellular response after vaccination is enhanced by directing the antigens of ASFV to the antigen-presenting cells by means of their fusion to the sHA fraction.

Such results allow concluding that the antibody and cellular response specifically directed against the antigens of ASFV after DNA vaccination can be improved if they are fused to the sHA fraction of ASFV.

## Claims

1. A gene construct comprising:
a) a polynucleotide (A) selected from:
(i) a polynucleotide comprising the nucleotide sequence encoding the hemagglutinin (HA) of African swine fever virus (ASFV);
(ii) a functionally equivalent fragment of said polynucleotide defined in (i); and
(iii)a functionally equivalent variant of said polynucleotide defined in (i) showing a minimum identity of 80% with respect to the encoding sequence of the HA of ASFV; and
b) a polynucleotide (B) comprising the nucleotide sequence encoding an antigen.

2. The gene construct according to claim 1, wherein the 3' end of said polynucleotide (A) is bound to the 5' end of said polynucleotide (B).

3. The gene construct according to claim 1, wherein the 5' end of said polynucleotide (A) is bound to the 3' end of said polynucleotide (B).

4. The gene construct according to any of claims 2 or 3, wherein said polynucleotide (A) comprises the nucleotide sequence encoding the soluble fraction of the hemagglutinin (sHA) of African swine fever virus (ASFV).

5. The gene construct according to any of claims 1 to 4, wherein said polynucleotide (A) comprises the nucleotide sequence encoding the protein the amino acid sequence of which is shown in SEQ ID NO: 1 or the nucleotide sequence encoding the protein the amino acid sequence of which is shown in SEQ ID NO: 2.

6. The gene construct according to any of claims 1 to 5, wherein the polynucleotide (B) encodes a fusion protein comprising the p54 and p30 proteins of ASFV.

7. The gene construct according to claim 6, wherein the polynucleotide (B) encodes a fusion protein comprising the amino acid sequence shown in SEQ ID NO: 3.

8. The gene construct according to claim 1, 4, 5, 6 or 7, comprising the nucleotide sequence shown in SEQ ID NO: 5.

9. A vector comprising a gene construct according to any of claims 1 to 8.

10. A fusion protein obtainable by the expression of the gene construct according to any of claims 1 to 8, or of a vector according to claim 9.

11. A fusion protein comprising:
a) a polypeptide (A') selected from:
(i) a polypeptide comprising the amino acid sequence of the hemagglutinin (HA) of African swine fever virus (ASFV);
(ii) a functionally equivalent fragment of said polypeptide defined in (i); and
(iii) a functionally equivalent variant of said polypeptide defined in (i) showing a minimum identity of 80% with respect to the amino acid sequence of the HA of ASFV; and
b) a polypeptide (B') comprising the amino acid sequence of an antigen.

12. The protein according to claim 11, wherein the carboxyl end of said polypeptide (A) is bound to the amino end of said polypeptide (B).

13. The protein according to claim 11, wherein the amino end of said polypeptide (A') is bound to the carboxyl end of said polypeptide (B').

14. The protein according to any of claims 11 to 13, wherein said polypeptide (A') comprises the soluble fraction of the hemagglutinin (sHA) of African swine fever virus (ASFV).

15. The protein according to any of claims 11 to 14, wherein said polypeptide (A') comprises the amino acid sequence shown in SEQ ID NO: 1 or the amino acid sequence shown in SEQ ID NO: 2.

16. The protein according to any of claims 11 to 15, wherein the polypeptide (B') is a fusion protein comprising the p54 and p30 proteins of ASFV.

17. The protein according to claim 16, wherein said protein has the amino acid sequence shown in SEQ ID NO: 4.

18. A host cell containing a gene construct according to any of claims 1 to 8, a vector according to claim 9, or a protein according to any of claims 10 to 17.

19. A host cell according to claim 18, wherein said cell is an antigen-presenting cell (APC).

20. A method for producing a fusion protein according to any of claims 10 to 17, comprising introducing in a host cell a gene construct according to any of claims 1 to 8, or a vector according to claim 9, and recovering the fusion protein from the culture medium or from the inside of the host cell.

21. A pharmaceutical composition comprising a therapeutically effective amount of a gene construct according to any of claims 1 to 8, or of a vector according to claim 9, or of a fusion protein according to any of claims 10 to 17, or of a host cell according to any of claims 18 or 19, together with a pharmaceutically acceptable carrier.

22. A use of a gene construct according to any of claims 1 to 8, or of a vector according to claim 9, or of a fusion protein according to any of claims 10 to 17, or of a host cell according to any of claims 18 or 19, in the preparation of a pharmaceutical composition for the treatment or prevention of a disease caused by an organism expressing or containing said antigen.

23. A use of the hemagglutinin (HA) of African swine fever virus (ASFV), or of a functionally equivalent fragment thereof, or of a functionally equivalent variant thereof showing a minimum identity of 80% with respect to the amino acid sequence of the HA of ASFV, as an adjuvant in the preparation of a pharmaceutical composition.

24. A use of the hemagglutinin (HA) of African swine fever virus (ASFV), or of a functionally equivalent fragment thereof, or of a functionally equivalent variant thereof showing a minimum identity of 80% with respect to the amino acid sequence of the HA of ASFV, as an adjuvant in the preparation of a composition for enhancing the immune response against an antigen jointly administered with said immune response-enhancing composition.

25. An antibody with the capacity to bind to a fusion protein according to any of claims 10 to 17.

26. A pharmaceutical composition comprising an antibody according to claim 25.

27. A use of an antibody according to claim 25 in the preparation of a pharmaceutical composition for the prevention and/or treatment of a disease caused by an organism containing the antigen present in said fusion protein.

28. An isolated cell of the immune system, with the capacity to recognize a fusion protein according to any of claims 10 to 17.

29. A pharmaceutical composition comprising a cell of the immune system according to claim 28.

30. A use of a cell of the immune system according to claim 28 in the preparation of a pharmaceutical composition for the prevention and/or treatment of a disease caused by an organism containing the antigen present in said fusion protein.
